# EUROPEAN PATENT SPECIFICATION

(11) **EP 2 806 922 B1**
(45) Date of publication and mention of the grant of the patent: **19.09.2018**
(21) Application number: 13701112.8
(22) Date of filing: 25.01.2013
(51) Int. Cl.: A61M 5/24, A61M 5/28

(54) **DRUG DELIVERY DEVICE WITH CARTRIDGE FIXATION FEATURE**
MEDIKAMENTENABGABEVORRICHTUNG MIT KARTUSCHENBEFESTIGUNGSFUNKTION
DISPOSITIF D'ADMINISTRATION DE MÉDICAMENT ÉQUIPÉ D'UN ÉLÉMENT DE FIXATION POUR CARTOUCHE

(30) Priority: 25.01.2012 EP 12152496; 30.01.2012 US 201261592078 P; 24.02.2012 EP 12156903; 28.02.2012 US 201261604181 P
(43) Date of publication of application: 03.12.2014
(73) Proprietor: Novo Nordisk A/S, 2880 Bagsværd (DK)
(72) Inventor: SØRENSEN, Morten, DK-2750 Ballerup (DK); WINDUM, Jesper Peter, DK-3400 Hillerød (DK); MADSEN, Kalle Holck, DK-2880 Bagsværd (DK); PLAMBECH, Christian, DK-2880 Bagsværd (DK); WINTHER, Knud Skifter, DK-2880 Bagsværd (DK)
(86) International application number: PCT/EP2013/051454
(87) International publication number: WO 2013/110769

(56) References cited:
- EP-A2- 0 937 474
- WO-A1-00/02605
- WO-A1-2004/020026
- WO-A1-2008/062025
- WO-A1-2011/039228
- WO-A1-2011/092326
- WO-A1-2011/124631
- US-A1- 2006 089 593

## Description

The present invention generally relates to a drug delivery device adapted to receive a drug filled cartridge and expel a dose therefrom.

### BACKGROUND OF THE INVENTION

In the disclosure of the present invention reference is mostly made to the treatment of diabetes, however, this is only an exemplary use of the present invention.

The most common type of injection devices adapted to receive a drug filled cartridge (also termed reservoir or container) and expel a dose therefrom are generally pen-formed and utilizes a so-called cartridge holder adapted to receive and mount a cartridge in the device. Correspondingly, most pen-formed drug delivery devices comprises a generally cylindrical cartridge holder for receiving and holding a generally cylindrical drug-filled cartridge in a mounted position, the cartridge comprising a proximally facing and axially displaceable piston, and a main body with a housing in which a drug expelling mechanism is arranged, the mechanism comprising an axially displaceable piston rod adapted to engage the piston of a mounted cartridge to thereby expel a dose of drug from the cartridge. Between the cartridge holder and the main body coupling means is provided allowing a user to remove the cartridge holder from the main body and reattach it when a used cartridge has been exchanged with a new cartridge, e.g. as shown in WO 2011/039228. The cartridge is inserted in the cartridge holder by axial movement through a proximal opening. The coupling means may be in the form of a threaded connection or a bayonet coupling. Depending on the design of the drug delivery device the piston rod in a durable device has to be moved proximally (i.e. "reset") by rotation when an empty cartridge is exchanged with a full cartridge, or the piston rod can be reset by being pushed axially, e.g. by unlocking the piston rod when the cartridge holder is removed from the main body, this as disclosed in e.g. US 2009/0275914 and WO 2011/051366. WO 2004/020026, upon which the two-part form of claim 1 is based, discloses a pen device comprising a front loaded cartridge holder in which the cartridge is prevented from moving distally by a pair of user-operated gripping arms. WO 2011/ 039228 discloses a cartridge with a distal coupling for a needle assembly, the cartridge being adapted for mounting in a cartridge holder.

Whereas the cartridge holder and the interface with the main body can be manufactured with narrow tolerances, the cartridges in most cases comprise a main cylindrical body manufactured from glass and thus inherently have very wide tolerances. Correspondingly, the parts of a drug delivery device adapted to receive and interface with such a cartridge has to allow relatively large tolerances of the glass part, especially lengthwise, yet providing functionality and dosing accuracy which is not influenced by length variations of the cartridge. To provide this most devices are designed to axially fixate a received cartridge.

Some of the reasons for providing a mechanism that safely fixes a user exchangeable cartridge part to a reusable (durable) part are: (1) if the exchangeable part is not safely axially fixed to the reusable part it may rattle which may lead to reduced perceived quality and possibly increases the risk of cracks and breakage of the drug cartridge. (2) If the exchangeable part is not safely axially fixed to the reusable part it may be possible for the user to push the drug cartridge towards the expelling mechanism in the reusable part of the device. Depending on the design of the expelling mechanism such a movement may lead to unintended out-dosing of drug.

This issue has been addressed in a number of ways in the past. For example, the drug delivery device may be provided with mechanical stop surfaces that restrict distal movement of the exchangeable part, and a spring that makes it difficult to push the exchangeable part in the proximal direction, however, this design still makes it possible to move the exchangeable part in the proximal direction if the user exceeds the spring force. This design is utilized in e.g. HumaPen® from Lilly. The issue regarding the risk of unintended out-dosing can be reduced by a relatively large spring force, just as when the user (correctly) mounts a needle assembly, the possible proximal movement of the exchangeable part can be restricted due to stop surfaces between the needle assembly hub and the cartridge holder. However if the user only mounts the needle assembly partially, it is still possible to perform an unintended out-dosing due to proximal movement of the drug cartridge. Another method of reducing the user's possibility to move the drug cartridge is by means of mechanical stop surfaces that restrict distal movement of the exchangeable part, and a transparent cartridge holder with no openings that encapsulates the drug cartridge. This design reduces the user's possibility to push the drug cartridge in the proximal direction, however, it does not eliminate the issues about rattling of the cartridge. This design is utilized in e.g. the ClikStar® pen device from Sanofi Aventis.

WO 2011/067269 discloses a further alternative in which axial movement of the drug cartridge is restricted by means of a snap interface between the cartridge and the cartridge holder, which engages the drug cartridge corresponding to its neck portion. For a durable device this solution requires that the user presses the cartridge onto the snap, and that the user can de-snap the cartridge before exchanging it. WO 2011/092326 discloses a cartridge holder comprising a coupling which engages and axially fixes a loaded cartridge when a needle assembly is mounted on the cartridge holder.

WO 2008/062025 discloses a yet further alternative in which axial movement of the drug container is restricted by means of a cartridge assembly in which the cartridge part and the cartridge holder with the coupling means are provided as a single disposable unit. The cartridge assembly may comprise a conventional glass cartridge enclosed in a polymeric enclosure or the cartridge assembly may have a generally integral design formed from a polymeric material. The latter type of design is also disclosed in WO 00/02605. In the OptiClik® pen device from Sanofi a disposable cartridge assembly is used in which also the piston rod is provided as part of the cartridge assembly.

Having regard to the above, it is an object of the present invention to provide a drug delivery device adapted to receive and hold a drug-filled cartridge in a simple and effective way, the arrangement being user-friendly, cost-effective and reliable.

### DISCLOSURE OF THE INVENTION

In the disclosure of the present invention, embodiments and aspects will be described which will address one or more of the above objects or which will address objects apparent from the below disclosure as well as from the description of exemplary embodiments.

Thus, in accordance with the invention a drug delivery system is provided comprising a cartridge, a cartridge holder and an expelling assembly, the cartridge comprising a cylindrical body portion having opposed distal and proximal portions, an axially displaceable piston arranged in the body portion, a distal outlet portion, a drug-filled variable volume reservoir being provided by the body portion and the piston, and first coupling means arranged at the distal portion. The cartridge holder is front-loaded and adapted to axially receive and hold the cartridge in a loaded position, and comprises a distal portion with a distal opening adapted to receive the cartridge in a proximal direction (i.e. the proximal portion of the cartridge is inserted through the cartridge holder distal opening in a proximal direction), and second coupling means arranged at the distal portion, wherein the first and second coupling means are configured to engage each other to thereby axially lock the received cartridge in the cartridge holder. The first and second coupling means are configured as cooperating rotational coupling means allowing a cartridge to be secured to the cartridge holder by relative rotational movement there between. The expelling assembly is adapted to engage and axially displace the piston in a loaded cartridge in a distal direction to thereby expel a dose of drug from the cartridge. The expelling assembly may be arranged in a housing providing an outer shell of a drug delivery device or it may be in the form of an assembly formed integrally with a housing. The cartridge holder may be formed integrally with the housing or be attached.

By this arrangement one or more of the following advantages can be achieved: Relatively easy integration of the coupling parts, no rattling of the cartridge in the cartridge holder, no risk of unintended out-dosing due to proximal movement of the cartridge, allows large production tolerances of the drug cartridge (e.g. a glass cartridge may actually be manufactured having larger tolerances than used today, this potentially saving costs), no need for a spring to make automatic adaption to the drug cartridge, possible to make an easy and intuitive solution for the user for attaching and releasing the cartridge, as well as enabling mechanical coding between the reusable part and the exchangeable part.

The cartridge may be provided with a needle interface allowing a needle assembly to be mounted in fluid communication with the reservoir. Alternatively the needle interface may be formed as part of the cartridge holder.

The cartridge may comprise a body part forming the cylindrical body portion with the needle interface and the first coupling means being attached at the distal outlet portion, e.g. in the form of an integral coupling part forming the needle interface and the first coupling means. The body part may comprise a distal neck portion with an opening closed by a needle penetrable septum, the integral coupling part being attached corresponding to the neck portion. The septum may be secured to the neck portion by a circumferential member to which the integral coupling part is attached. Alternatively, the cylindrical body portion, the needle interface and the first coupling means may be formed integrally from a polymeric material.

In a shorter wording the present invention provides a drug delivery system comprising a drug-filled cartridge, a drug delivery device with a front-loaded cartridge holder adapted to axially receive and hold the cartridge in a loaded position, the cartridge holder comprising a distal opening adapted to receive the cartridge in a proximal direction, wherein the cartridge and the cartridge holder are provided with coupling means configured to engage each other to thereby axially lock the received cartridge in the cartridge holder. The cartridge and drug delivery device may comprise additional specific features as disclosed above.

As used herein, the term "drug" is meant to encompass any flowable medicine formulation capable of being passed through a delivery means such as a cannula or hollow needle in a controlled manner, such as a liquid, solution, gel or fine suspension, and containing one or more drug agents. The drug may be a single drug compound or a premixed or co-formulated multiple drug compounds drug agent from a single reservoir. Representative drugs include pharmaceuticals such as peptides (e.g. insulins, insulin containing drugs, GLP-1 containing drugs as well as derivatives thereof), proteins, and hormones, biologically derived or active agents, hormonal and gene based agents, nutritional formulas and other substances in both solid (dispensed) or liquid form. In the description of the exemplary embodiments reference will be made to the use of insulin and GLP-1 containing drugs, this including analogues thereof as well as combinations with one or more other drugs.

### BRIEF DESCRIPTION OF THE DRAWINGS

In the following the invention will be further described with reference to the drawings, wherein
fig. 1 shows an embodiment of a drug delivery device,
figs. 2A and 2B show a schematic representation of a front loaded drug delivery device,
figs. 3A and 3B show a first embodiment of a front loaded cartridge and cartridge holder assembly,
figs. 4A and 4B show a second embodiment of a front loaded cartridge and cartridge holder assembly,
figs. 5A and 5B show a third embodiment of a front loaded cartridge and cartridge holder assembly,
fig. 6 show fourth embodiment of a front loaded cartridge and cartridge holder assembly,
figs. 7A and 7B show schematically an embodiment of a drug delivery device with spring means for moving a piston rod into a loading position,
figs. 8A and 8B show schematically a further embodiment of a drug delivery device with spring means for moving a piston rod into a loading position,
figs. 9A and 9B show schematically a yet further embodiment of a drug delivery device with spring means for moving a piston rod into a loading position,
figs. 10A and 10B show schematically a yet further embodiment of a drug delivery device with spring means for moving a piston rod into a loading position,
figs. 11A-11C show schematically an embodiment of a drug delivery device with coupling means for moving a piston rod into a loading position, and
figs. 12A-12C show schematically a further embodiment of a drug delivery device with coupling means for moving a piston rod into a loading position.

In the figures like structures are mainly identified by like reference numerals.

### DESCRIPTION OF EXEMPLARY EMBODIMENTS

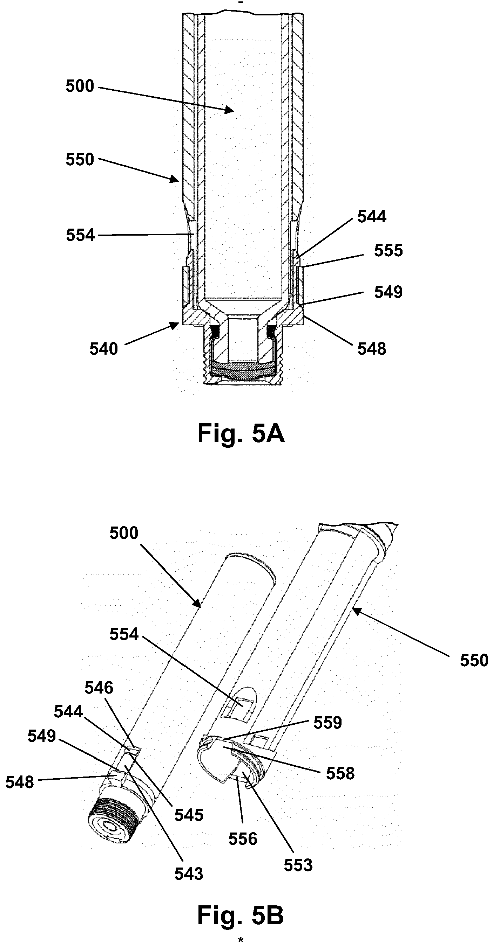

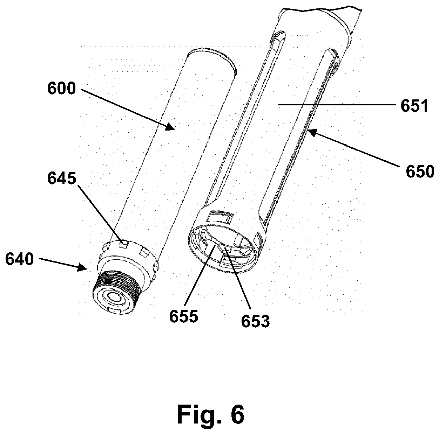

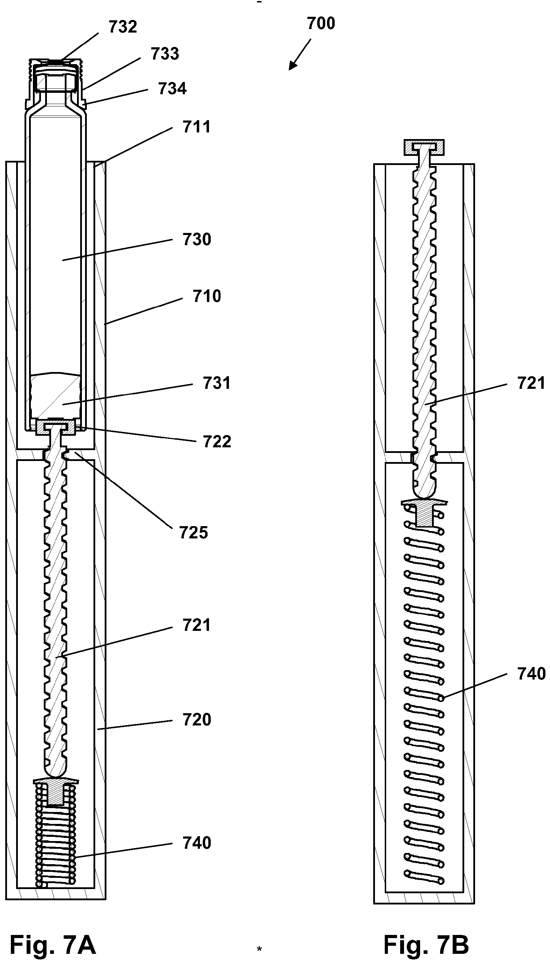

When in the following terms such as "upper" and "lower", "right" and "left", "horizontal" and "vertical" or similar relative expressions are used, these only refer to the appended figures and not to an actual situation of use. The shown figures are schematic representations for which reason the configuration of the different structures as well as their relative dimensions are intended to serve illustrative purposes only. Figs. 5A, 5B and 6 disclose embodiments outside the scope of the invention as claimed. Figs. 7A to 12C disclose embodiments comprising features which may be useful in combination with the present invention as claimed.

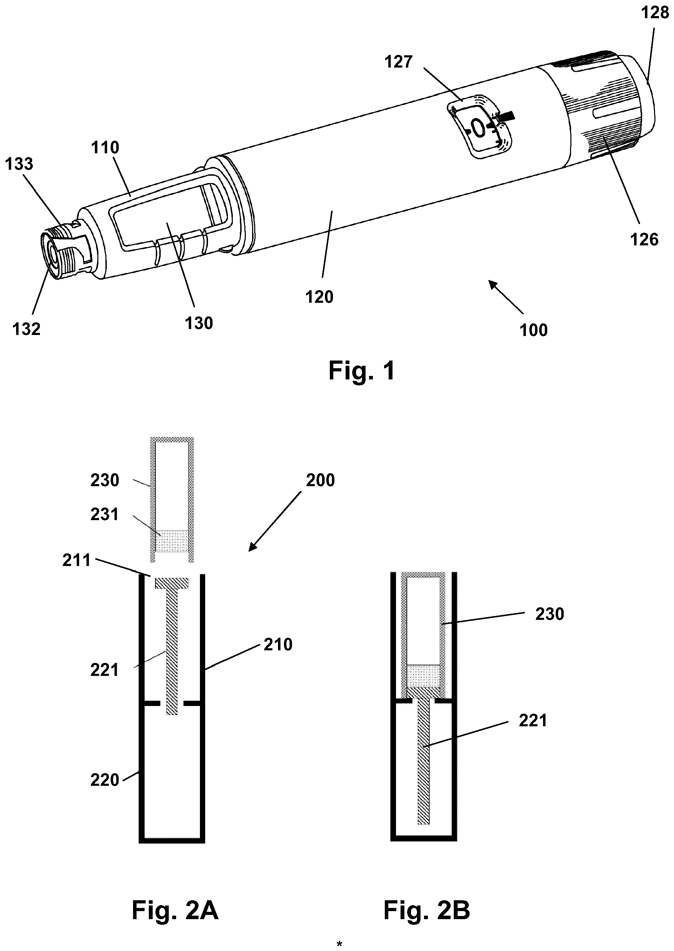

Referring to fig. 1 a pen-formed drug delivery device 100 will be described. The device represents a "generic" drug delivery device providing an example of a device in combination with which embodiments of the present invention is intended to be used. More specifically, the pen device comprises a cap part (not shown) and a main part having a proximal body portion 120 in which a drug expelling mechanism is arranged or integrated, and a distal cartridge holder portion in which a drug-filled generally transparent cartridge 130 with a distal needle-penetrable septum 132 is arranged and held in place by a cartridge holder 110 attached to the proximal portion, the cartridge holder having openings allowing a portion of the cartridge to be inspected. The device is designed to be loaded by the user with a new cartridge through a distal receiving opening in the cartridge holder, the cartridge being provided with a piston driven by a piston rod forming part of the expelling mechanism. A proximal-most rotatable dose ring member 126 serves to manually set a desired dose of drug shown in display window 127 and which can then be expelled when the release button 128 is actuated. Depending on the type of drug delivery device, the expelling mechanism may comprise a spring which is strained during dose setting and then released to drive the piston rod when the release button is actuated. Alternatively the expelling mechanism may be fully manual in which case the dose ring member and the release button moves proximally during dose setting corresponding to the set dose size, and then moved distally by the user to expel the set dose. The cartridge is provided with distal coupling means in the form of a needle hub mount 133 having, in the shown example, an external thread as well as a bayonet adapted to engage an inner thread or a bayonet of a corresponding hub of a needle assembly (see below). The cartridge holder is adapted to receive and hold the cartridge in a loaded position, the holder having a generally tubular configuration with a distal opening adapted to axially receive the cartridge, the holder and the cartridge being provided with corresponding coupling means allowing a cartridge to be mounted and subsequently released. Different embodiments of such coupling means will be described in greater detail in the following. An example of an expelling mechanism allowing a user to set a desired dose as well as comprising a cartridge actuated coupling allowing the piston rod to be pushed back by a cartridge during loading is disclosed in e.g. US 2004/0210199 hereby incorporated by reference.

When using a drug delivery device of the above general type (which may have other form-factors and also be provided with a motorized expelling mechanism), the user is typically recommended to take a subcutaneous injection by performing the following steps: remove the cap to uncover the needle mount, mount a new needle assembly, set a dose amount to be expelled by rotating the dose setting member, when the needle has been inserted subcutaneously actuate the release means for driving or releasing the drug expelling means to expel the set dose, after having withdrawn the needle from the skin remove the needle assembly from the needle mount, and re-attach the cap to cover the needle mount.

Referring to figs. 2A and 2B a pen-formed drug delivery device of the front loading type is shown schematically. More specifically, fig. 1A shows a pen-formed drug delivery device 200 comprising a generally cylindrical cartridge holder 210 for receiving and holding a generally cylindrical drug-filled cartridge 230 in a mounted position, the cartridge comprising a proximally facing and axially displaceable piston 231 as well as a distal septum (not shown) allowing a subcutaneous needle assembly to be connected in fluid communication with the interior of the cartridge which for example may contain an insulin, GLP-1 or growth hormone formulation. The needle assembly is connected via coupling means provided as part of the cartridge. The cartridge holder comprises a distal opening 211 for receiving a cartridge and is attached to or integrated with a main body 220 comprising a housing in which a drug expelling mechanism is arranged or integrated, the mechanism comprising an axially displaceable piston rod 221 adapted to engage the piston of a mounted cartridge to thereby expel a dose of drug from the cartridge, e.g. a user set dose. The expelling mechanism may e.g. be driven manually, by a spring energized during dose setting, or by electric means. The cartridge and the cartridge holder are provided with cooperating locking means (not shown, see below) allowing a cartridge to be inserted and releasable held in place in the cartridge holder as shown in fig. 1B. The expelling mechanism is provided with coupling means (not shown) acting directly or indirectly on the piston rod and being actuatable between an operational state in which the piston rod can be moved distally to expel a dose of drug from the cartridge, and a loading state in which the piston rod can be moved proximally by e.g. axial movement when a new cartridge is inserted, the piston of the cartridge engaging and pushing the piston rod proximally. The piston rod coupling means may be operated between its loading and operational state (and vice versa) when e.g. a cartridge is inserted as disclosed in US 2004/ 0210199, when the cartridge locking means is operated or by separate additional user operateable means.

Different alternatives to provide the desired coupling functionality between the cartridge and the cartridge holder will be described with reference to embodiments of a cartridge holder in combination with a drug-filled cartridge, the figures not showing details in respect of the remaining drug delivery device.

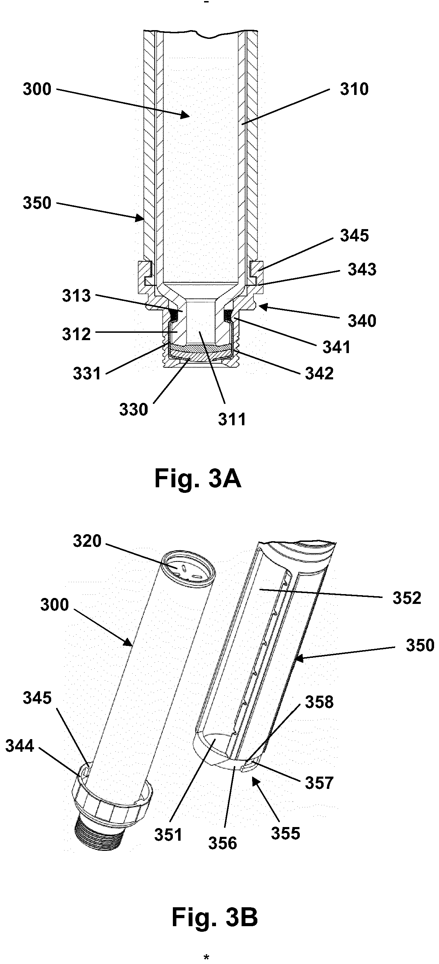

Figs. 3A and 3B show an embodiment of an assembly comprising a cartridge 300 and a cartridge holder 350 adapted to receive the cartridge in locking engagement. The cartridge comprises a glass main reservoir part having a cylindrical body portion 310 in which an axially displaceable polymeric piston 320 is arranged, the glass part comprising a distal outlet portion with an outlet opening 311 surrounded by a circumferential flange 312, and a reduced-diameter neck portion 313 connecting the body and outlet portions. The opening is closed by a needle penetrable septum 330 (shown as a laminate) attached to and held in sealed engagement with the glass part by a circumferential ring member 331 swaged around the flange. The cartridge is further provided with a non-releasable coupling member 340 mounted on the ring member and held in gripping engagement by means of a number of inwardly oriented protrusions 341 preventing axial movement between the coupling member and the glass part. The coupling member may or may not be allowed to rotate relative to the glass main part. The coupling member is provided with a threaded needle interface 342 allowing a needle assembly to be mounted in fluid communication with the reservoir, as well as coupling means adapted to engage corresponding coupling means on the cartridge holder. In the figs. 3A and 3B embodiment the coupling member is provided with a proximally facing skirt portion 343 providing a circumferential slot 344 between the skirt portion and the cylindrical body portion, the slot being adapted to receive the distal portion of the cartridge holder, the skirt portion being provided with the coupling means in the form of a pair of opposed inwardly oriented protrusions 345. The cartridge holder 350 has a general cylindrical configuration with a distal opening 351 for receiving the cartridge as well as a pair of opposed windows 352 allowing a mounted cartridge to be inspected by the user. On the outer distal circumference of the cartridge holder coupling means in the form of a pair of opposed slots 355 are arranged, each slot having a distal opening 356 adapted to axially receive the above-described skirt protrusions, the remaining portion 357 of the slot being adapted to accommodate the protrusion when the coupling member is rotated relative to the cartridge holder, thereby providing a bayonet coupling. In the shown embodiment the closed portion of the slot has a length corresponding approximately to 5 degrees of the cartridge holder circumference. The "floor" of the slot is provided with a small ridge 358, this allowing the skirt protrusion to snap in place due to the flexibility of the skirt portion. As appears, when the cartridge has been inserted axially and locked into its mounted position by a small additional rotation, the cartridge is locked axially in the cartridge holder.

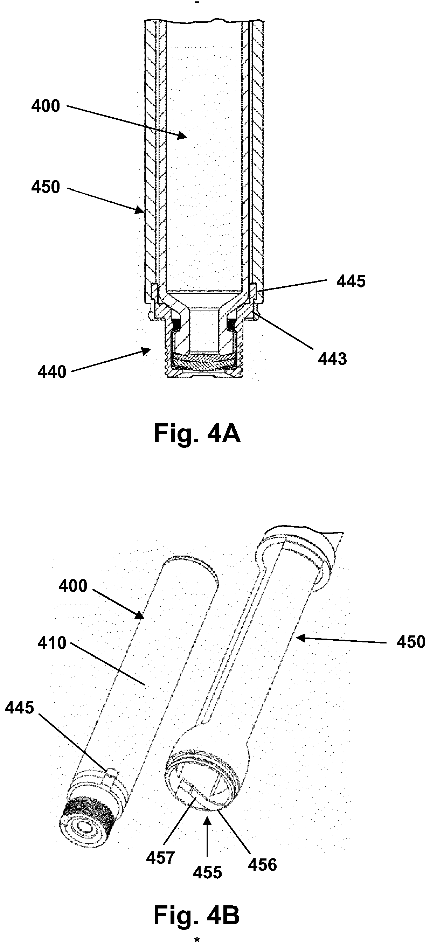

Figs. 4A and 4B show a further embodiment of an assembly comprising a cartridge 400 with a coupling member 440 and a cartridge holder 450 adapted to receive the cartridge in locking engagement. The assembly corresponds to the above-described embodiment with the main difference that the bayonet coupling is provided between an outer surface of the cartridge skirt and an inner surface of the cartridge holder. More specifically, the skirt portion 443 is provided with coupling means in the form of a pair of opposed outwardly oriented protrusions 445. The cartridge holder 450 has on the inner distal surface coupling means in the form of a pair of opposed slots 455, each slot having a distal opening 456 adapted to axially receive the above described skirt protrusions, the remaining portion 457 of the slot being adapted to accommodate the protrusion when the coupling member is rotated relative to the cartridge holder, thereby providing a bayonet coupling.

Figs. 5A and 5B show a further embodiment of an assembly comprising a cartridge 500 and a cartridge holder 550 adapted to receive the cartridge in locking engagement. The assembly corresponds to the above-described embodiment with the main difference that the coupling is an axially actuated snap coupling comprising flexible fingers arranged on the cartridge. More specifically, the coupling member 540 is provided with coupling means in the form of a pair of opposed proximally extending flexible fingers 543 each comprising a proximal outwards protrusion 544 with a distally facing edge 545 and a proximally facing inclined surface 546. Distally of each finger a distal protrusion 548 with a proximally facing edge 549 is arranged. The cartridge holder 550 has on the inner distal surface coupling means in the form of a pair of opposed axially extending slots 553, each slot having a distal opening adapted to axially receive the above-described skirt protrusions, each slot communicating at the proximal end with an opening 554 adapted to receive a skirt protrusion, each opening having a proximally facing edge 555. Corresponding to each slot the distal circumferential edge of the cartridge holder is provided with a pair of cut-outs 558 each having a distally facing edge 559 with an associated inclined surface 556 adapted to initially engage the inclined surface 546 on a flexible arm. When the cartridge is axially inserted in the cartridge holder the fingers engages the slots and flexes inwardly until the finger protrusions snap outwardly into the openings, the distal edge on each protrusion thereby facing the corresponding proximal edge of the opening. At the same time the cartridge distal protrusions 548 seat into the corresponding cut-outs 558, the proximal edge 549 on each distal protrusion thereby facing the corresponding distal edge 559 of the cut-out. In this way the cartridge is locked in the cartridge holder both axially and rotationally. To release the cartridge holder the user forces the two finger protrusions inwardly after which the cartridge can be axially removed from the cartridge holder.

Fig. 6 show a further embodiment of an assembly comprising a cartridge 600 and a cartridge holder 650 adapted to receive the cartridge in locking engagement. The assembly corresponds to the above-described embodiment with the main difference that the coupling has an open/close interface actuated by a user operated locking component arranged on the cartridge holder. More specifically, the coupling member 640 is provided with coupling means in the form of a plurality of circumferentially arranged outwards protrusions 645 adapted to be axially received in corresponding seats in the cartridge holder.

The cartridge holder 650 comprises a body portion on which a rotatable sleeve 651 is arranged. The body portion comprises on the inner distal surface coupling means in the form of a plurality of projections 653 providing a plurality of spaces 655 in which the cartridge projections can seat when the cartridge is inserted axially in the cartridge holder with the sleeve in its "open" rotational position. When the sleeve is rotated to its "closed" position the cartridge projections are locked both axially and rotationally in their seated position.

The above-described embodiments of figs. 3-6 comprise a threaded interface for a needle assembly, however, the interface could also be provided by a bayonet coupling or a combined thread and bayonet coupling as shown in fig. 1. Correspondingly, the embodiments of figs. 3-6 comprise a cartridge holder with a pair of windows allowing a user to inspect an inserted cartridge, however, alternatively the cartridge holder could be made from a transparent material without openings. In the shown embodiments the cartridge comprises a main glass reservoir part to which a coupling member is attached, however, for a polymeric reservoir part the coupling interfaces could be integrated fully or partly therewith. Further, the cartridge and/or the means for receiving and holding the cartridge in a loaded position may be provided with non-releasable coupling means, this allowing the system to be used for the manufacture of a pre-filled drug delivery device. An advantage of such a system would be that a drug-filled cartridge, which is normally the most expensive part of a pre-filled drug delivery device, could be inserted as one of the final steps of the manufacturing process.

To prevent that a given cartridge is mounted in a cartridge holder of a drug delivery device not specifically adapted for the contained drug formulation, a cartridge and a corresponding cartridge holder may be provided with corresponding key structures allowing the cartridge to be mounted in the cartridge holder, but preventing a cartridge with a different key structure to be mounted, the latter being adapted to be mounted in a correspondingly adapted other drug delivery device. The key structures may e.g. be a system of projections and receiving slots or grooves and be adapted to be received by relative axial or rotational movement or a combination thereof. The cartridge key structures may be formed integrally with the coupling member.

Figs. 7A and 7B show in a schematic representation an embodiment of a front loaded pen-formed drug delivery device, fig. 7A showing the device with a loaded cartridge and fig. 7B showing the device when the cartridge has been removed. More specifically, fig. 7A shows a pen-formed drug delivery device 700 comprising a generally cylindrical cartridge holder 710 for receiving and holding a generally cylindrical drug-filled cartridge 730 in a mounted position, the cartridge comprising a proximally facing and axially displaceable piston 731 as well as a distal septum 732 and a coupling member 733 allowing a subcutaneous needle assembly to be connected in fluid communication with the interior of the cartridge. The coupling member is further provided with coupling means 734 adapted to engage corresponding coupling means (not shown) on the cartridge holder allowing a cartridge to be inserted and releasable hold in place in the cartridge holder, however, the cartridge may be mounted in the cartridge holder by any suitable means, e.g. as shown in WO 2004/020026. The cartridge holder comprises a distal opening 711 for receiving the cartridge and is in the shown embodiment integrated with a main body 720 comprising a housing in which a drug expelling mechanism is arranged, the mechanism comprising an axially displaceable piston rod 721 with a washer 722 adapted to engage the piston of a mounted cartridge to thereby expel a dose of drug from the cartridge, e.g. a user set dose. The piston rod is in a non-locking threaded engagement with a correspondingly threaded nut member 725 of the expelling mechanism (here schematically shown as a part of the housing), whereby the piston rotates as it is moved proximally and distally.

In the figures only the piston rod is shown. The expelling mechanism may e.g. be driven manually, by a spring energized during dose setting, by a pre-loaded spring or by electrically driven means. The expelling mechanism is provided with coupling means (not shown) acting directly or indirectly on the piston rod and being actuatable between an operational state in which the piston rod can be moved distally to expel a dose of drug from the cartridge, and a loading state in which the piston rod can be moved proximally by e.g. axial movement when a new cartridge is inserted, the piston of the cartridge engaging and pushing the piston rod proximally. The piston rod coupling means may be operated between its loading and operational state (and vice versa) when e.g. a cartridge is inserted (see e.g. US 2004/0210199 which discloses an example of a coupling in which a driving member rotating the piston rod during expelling of a dose can be de-coupled from the expelling mechanism), when the cartridge locking means is operated or by separate additional user operateable means.

In order to ensure that there is no gap between the piston rod and the piston when a new cartridge has been fully inserted and locked in place, the drug delivery device is provided with additional spring means such that the piston rod automatically is moved to its distal loading position when a cartridge is removed from a loaded position and the piston rod thereby un-locked. More specifically, a helical spring 740 is provided providing a distally directed biasing force on the piston rod, the spring being in a (fully or partly) compressed state when the piston rod as shown in fig. 7A is positioned in the loaded position with a full cartridge mounted in the cartridge holder. When the cartridge is removed from the cartridge holder as shown in fig. 7B the spring expands and pushes the un-coupled rotating piston rod distally to a distal-most position.

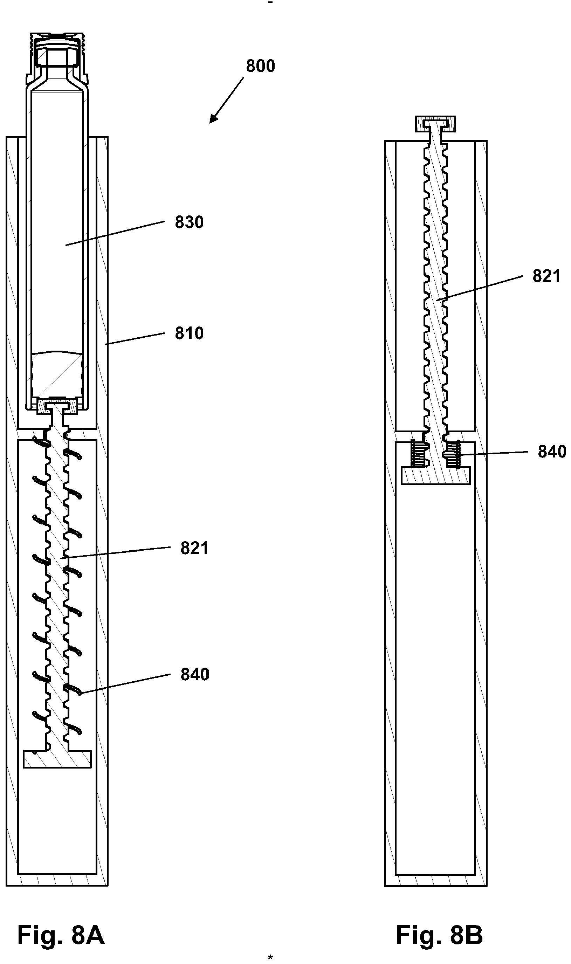

The embodiment of a drug delivery device 800 shown in figs. 8A and 8B corresponds to the embodiment of figs. 7A and 7B with the main difference that the spring 840 is arranged to be stretched with the piston rod 821 as shown in fig. 8A is positioned in the loaded position with a full cartridge 830 mounted in the cartridge holder 810. When the cartridge is removed from the cartridge holder as shown in fig. 8B the spring contracts and pushes the un-coupled piston rod distally to a distal-most position.

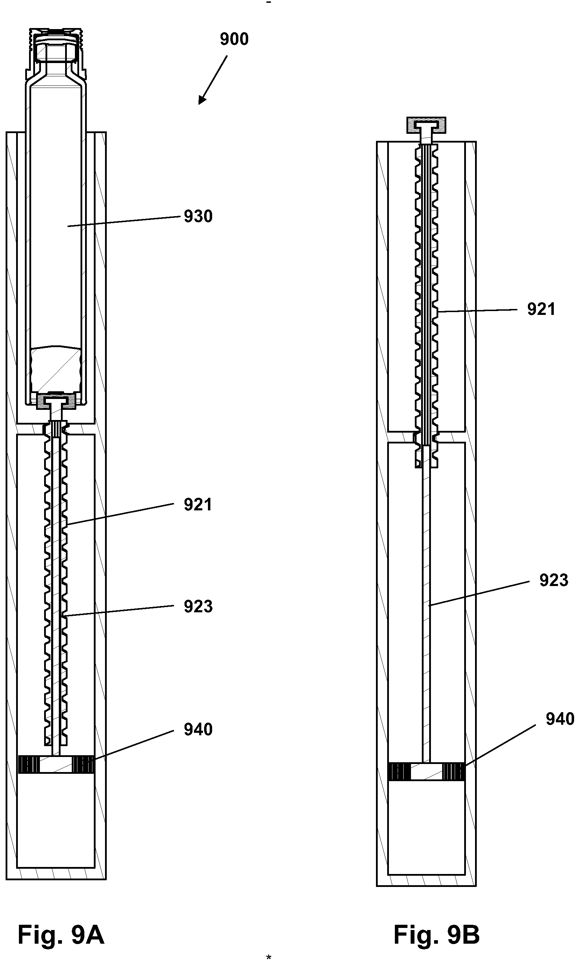

The embodiment of a drug delivery device 900 shown in figs. 9A and 9B is similar to the embodiment of figs. 7A and 7B with the main difference that the spring is a spiral torsion spring 940 providing torsion to a torsion rod 923 housed axially inside a hollow piston rod 921, a threaded connection being provided there between. When the cartridge 930 is removed from the cartridge holder as shown in fig. 9B the axially stationary torsion rod is rotated by the spring and the piston rod is correspondingly rotated and moved distally to its distal-most position.

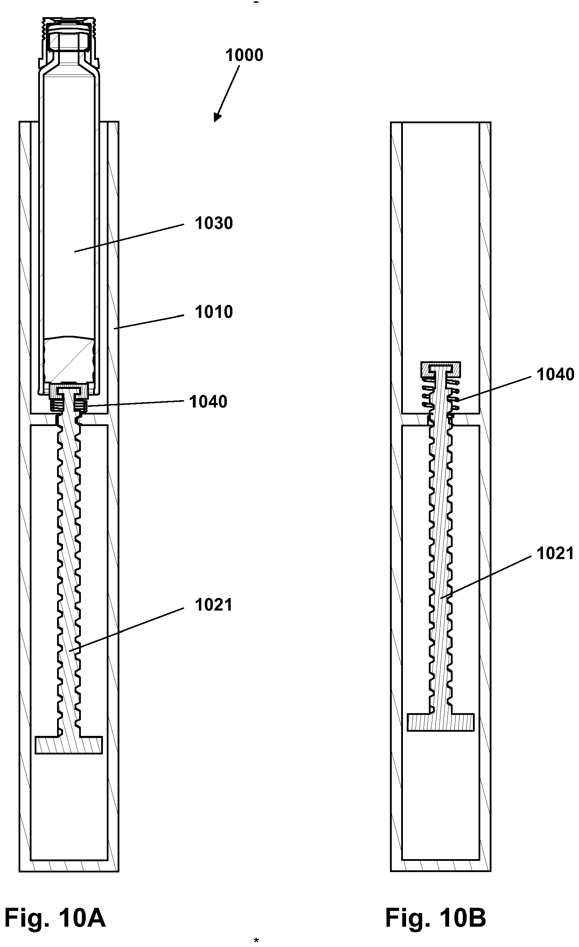

The embodiment of a drug delivery device 1000 shown in figs. 10A and 10B is similar to the embodiment of figs. 7A and 7B with the main difference that the coil spring 1040 is much shorter and arranged inside the cartridge holder 1010. Such a short spring will assure that the piston rod 1021 will always be positioned in a loading position corresponding to a full cartridge but will not assure that the piston rod will always engage the piston when a partly full cartridge is inserted. Such a design could also be used when assembling a pre-filled drug delivery device, the design assuring that there will be no air gap between the piston rod and the piston.

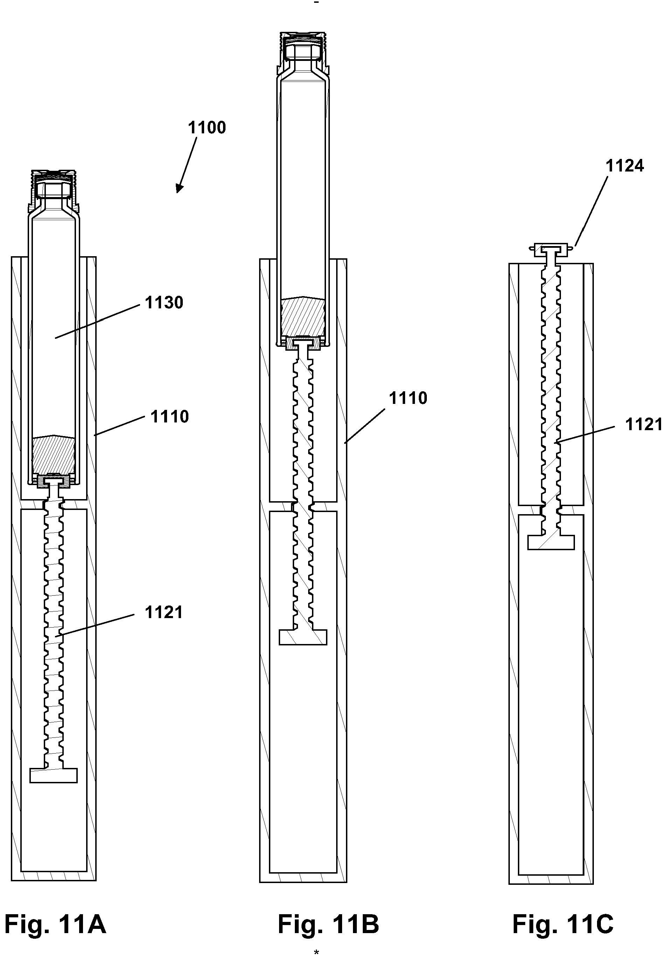

The embodiment of a drug delivery device 1100 shown in figs. 11A-11C differs from the embodiment of figs. 7A and 7B in that the piston rod 1121 is provided with a releasable coupling 1124 frictionally engaging the proximal-most portion of the interior cylinder surface of a cartridge. When removing the cartridge 1130 from cartridge holder 710 the un-locked piston rod is pulled distally by the cartridge, see fig. 11B. When the piston rod reaches its distal-most position the frictional coupling will disengage and the cartridge can be removed, see fig. 11C. When a new cartridge is inserted it will frictionally engage the piston rod coupling at the latest when the cartridge is pushed into its loaded proximal-most position.

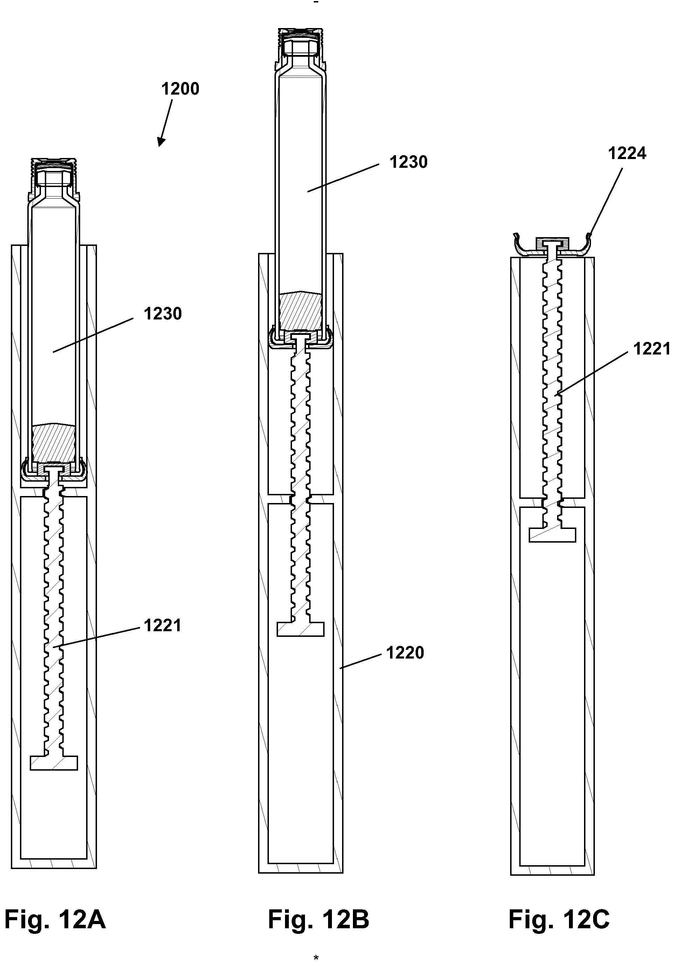

The embodiment of a drug delivery device 1200 shown in figs. 12A-12C corresponds to the embodiment of figs. 11A-11C with the main difference that the coupling 1224 is adapted to frictionally engaging the proximal-most portion of the exterior cylinder surface of a cartridge 1230. Otherwise the two embodiments functions essentially in the same way.

The above-described embodiments of a pen-formed drug delivery device are all adapted to receive a cartridge through a distal opening, i.e. they are of a front loaded design. However, the different designs for biasing or moving a piston rod to a distal loading position could also be utilized in combination with a rear-loaded cartridge holder. As an alternative the cartridge could be loaded sideways into a cartridge holder, e.g. as in some drug delivery devices having a "dozer" form factor such as Innovo® from Novo Nordisk.

In the above description of exemplary embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A drug delivery system comprising:
(a) a cartridge (300, 400, 500, 600) comprising:
- a cylindrical body portion (230) having opposed distal and proximal portions,
- an axially displaceable piston (231) arranged in the body portion,
- a distal outlet portion (311), and
- a drug-filled variable volume reservoir being provided by the body portion and the piston,
(b) a front-loaded cartridge holder (350, 450, 550, 650) adapted to axially receive and hold the cartridge in a loaded position, comprising:
- a distal portion with a distal opening (351) adapted to receive the cartridge in a proximal direction,
(c) an expelling assembly adapted to engage and axially displace the piston in a loaded cartridge in a distal direction to thereby expel a dose of drug from the cartridge,
**characterized in that**
- the cartridge comprises first coupling means (345, 445, 545, 645) arranged at the distal portion, and
- the front-loaded cartridge holder comprises second coupling means (355, 455, 555, 655) arranged at the distal portion,
wherein the first and second coupling means are configured to engage each other to thereby axially lock the received cartridge in the cartridge holder, and
wherein the first and second coupling means are configured as cooperating rotational coupling means (345, 355, 445, 455) allowing a cartridge to be secured to the cartridge holder by relative rotational movement there between.

2. A drug delivery system as in claim 1, wherein the cartridge comprises a needle interface (342) allowing a needle assembly to be mounted in fluid communication with the reservoir.

3. A drug delivery system as in claim 2, wherein the cartridge comprises a body part (310) forming the cylindrical body portion, the needle interface (342) and the first coupling (345) means being attached at the distal outlet portion.

4. A drug delivery system as in claim 3, wherein the cartridge comprises an integral coupling part (340, 440, 540, 640) forming the needle interface and the first coupling means.

5. A drug delivery system as in claim 3, wherein the body part comprises a distal neck portion (313) with an opening (311) closed by a needle penetrable septum (330), the integral coupling part (340) being attached to the neck portion.

6. A drug delivery system as in claim 6, wherein the septum (330) is secured to the neck portion by a circumferential member (331) to which the integral coupling part is attached.

7. A drug delivery system as in any of claims 2-6, wherein the cylindrical body portion, the needle interface and the first coupling means are formed integrally from a polymeric material.

8. A drug delivery system as in any of the previous claims, wherein the first and second coupling means are releasable allowing a mounted cartridge to be removed from the cartridge holder by a user.

9. A drug delivery system as in any of the previous claims, further comprising a housing (220) wherein the expelling assembly is at least partially arranged, the cartridge holder (210) being formed as part of the housing.

10. A drug delivery system as in any of the previous claims, wherein:
- the axially displaceable piston has an initial proximal position, a fully-forwarded distal position, and an intermediate position there between,
- the piston rod is moveable between a proximal loaded position and a distal-most position, a loading position being defined distally of the proximal position,
- the piston rod, when positioned proximally of the loading position, is moved to its loading position when a cartridge is removed from a loaded position, and
- the piston rod is adapted to engage a piston and to be moved proximally towards its loaded position when a cartridge is arranged in a loaded position.

11. A drug delivery system as in any of the previous claims, comprising a drug delivery device comprising the cartridge holder and the expelling assembly.

## Patentansprüche

1. Ein Medikamentenabgabesystem, umfassend:
(a) Eine Patrone (300, 400, 500, 600), umfassend:
- einen zylindrischen Gehäuseabschnitt (230) mit entgegengesetzten distalen und proximalen Abschnitten,
- einen axial verschiebbaren Kolben (231), der in dem Gehäuseabschnitt angeordnet ist,
- einen distalen Auslassabschnitt (311), und
- einen medikamentengefüllten Behälter mit variablem Volumen, der von dem Gehäuseabschnitt und dem Kolben bereitgestellt wird,
(b) einen von vorne beladenen Patronenhalter (350, 450, 550, 650), der angepasst ist, um die Patrone axial in einer geladenen Position zu empfangen und zu halten, umfassend:
- einen distalen Abschnitt mit einer distalen Öffnung (351), die angepasst ist, die Patrone in einer proximalen Richtung aufzunehmen,
(c) eine Ausstoßanordnung, die angepasst ist, sich mit dem Kolben in einer geladenen Patrone zu verbinden und axial in eine distale Richtung zu verschieben, um dadurch eine Dosis eines Medikaments aus der Patrone auszustoßen,
**dadurch gekennzeichnet, dass**.
- die Patrone eine erste Kopplungsvorrichtung (345, 445, 545, 645) umfasst, die im distalen Abschnitt angeordnet ist, und
- der von vorne geladene Patronenhalter eine zweite Kopplungsvorrichtung (355, 455, 555, 655) umfasst, die im distalen Abschnitt angeordnet ist,
wobei die erste und die zweite Kopplungsvorrichtung konfiguriert sind, sich miteinander zu verbinden, um dadurch die aufgenommene Patrone axial im Patronenhalter zu verriegeln, und
wobei die erste und die zweite Kopplungsvorrichtung als mitwirkende Drehkopplungsvorrichtungen (345, 355, 445, 455) konfiguriert sind, die es einer Patrone erlauben, an dem Patronenhalter durch eine Drehbewegung dazwischen befestigt zu werden.

2. Ein Medikamentenabgabesystem nach Anspruch 1, wobei die Patrone eine Nadel-Schnittstelle (342) umfasst, mit der eine Nadelanordnung in Fluidverbindung mit dem Behälter montiert werden kann.

3. Ein Medikamentenabgabesystem nach Anspruch 2, wobei die Patrone einen Gehäuseteil (310) umfasst, der den zylindrischen Gehäuseabschnitt bildet, wobei die Nadel-Schnittstelle (342) und Kopplungsvorrichtung (345) am distalen Auslassabschnitt befestigt sind.

4. Ein Medikamentenabgabesystem nach Anspruch 3, wobei die Patrone einen eingebauten Kopplungsteil (340, 440, 540, 640) umfasst, der die Nadel-Schnittstelle und die erste Kopplungsvorrichtung bildet.

5. Ein Medikamentenabgabesystem nach Anspruch 3, wobei der Gehäuseteil einen distalen Halsabschnitt (313) mit einer Öffnung (311) umfasst, die durch ein mit einer Nadel durchdringbares Septum (330) geschlossen ist, wobei der eingebaute Kopplungsteil (340) an dem Halsabschnitt befestigt ist.

6. Ein Medikamentenabgabesystem nach Anspruch 6, wobei das Septum (330) am Halsabschnitt durch ein Umlaufteil (331) befestigt ist, an dem wiederum das eingebaute Kopplungsteil befestigt ist.

7. Ein Medikamentenabgabesystem nach den Ansprüchen 2-6, wobei der zylindrische Gehäuseabschnitt, die Nadelschnittstelle und die erste Kopplungsvorrichtung vollständig aus einem polymeren Material geformt sind.

8. Ein Medikamentenabgabesystem nach einem der vorhergehenden Ansprüche, wobei die erste und die zweite Kopplungsvorrichtung abnehmbar sind und es einer eingesetzten Patrone erlauben, von einem Benutzer aus dem Patronenhalter entnommen zu werden.

9. Ein Medikamentenabgabesystem nach einem der vorhergehenden Ansprüche, ferner umfassend ein Gehäuse (220), in dem die Ausstoßanordnung mindestens teilweise angeordnet ist, wobei der Patronenhalter (210) als Teil des Gehäuses ausgebildet ist.

10. Ein Medikamentenabgabesystem nach einem der vorhergehenden Ansprüche, wobei:
- der axial verschiebbare Kolben eine anfängliche proximale Position, eine vollständig vorwärts gerichtete distale Position und eine Zwischenposition dazwischen aufweist,
- die Pleuelstange beweglich zwischen einer proximalen geladenen Position und einer am weitesten entfernten distalen Position ist, eine Ladeposition distal zu der proximalen Position definiert ist,
- die Pleuelstange, wenn sie proximal zur Ladeposition positioniert ist, in ihre Ladeposition bewegt wird, wenn eine Patrone aus einer geladenen Position entnommen wird, und
- die Pleuelstange angepasst ist, sich mit einem Kolben zu verbinden und proximal zu seiner geladenen Position bewegt zu werden, wenn eine Patrone in einer geladenen Position angeordnet ist.

11. Ein Medikamentenabgabesystem nach einem der vorhergehenden Ansprüche, das eine Medikamentenabgabevorrichtung umfasst, die den Patronenhalter und die Ausstoßanordnung umfasst.

## Revendications

1. Système d'administration de médicament comprenant :
(a) une cartouche (300, 400, 500, 600) comprenant :
- une partie de corps cylindrique (230) ayant des parties distale et proximale opposées,
- un piston (231) pouvant être déplacé axialement disposé dans la partie de corps,
- une partie sortie distale (311) et
- un réservoir à volume variable rempli de médicaments étant fourni par la partie de corps et le piston,
(b) un porte-cartouche à chargement frontal (350, 450, 550, 650) adapté pour recevoir axialement et maintenir la cartouche dans une position chargée, comprenant :
- une partie distale avec une ouverture distale (351) adaptée pour recevoir la cartouche dans une direction proximale,
(c) un assemblage d'expulsion adapté pour engager et déplacer axialement le piston dans une cartouche chargée dans une direction distale pour ainsi expulser une dose de médicament de la cartouche,
**caractérisé en ce que**
- la cartouche comprend un premier moyen de couplage (345, 445, 545, 645) agencé au niveau de la partie distale, et
- le porte-cartouche à chargement frontal comprend un second moyen de couplage (355, 455, 555, 655) disposé sur la partie distale,
où les premier et second moyens de couplage sont configurés pour venir en prise les uns avec les autres afin de verrouiller axialement la cartouche reçue dans le porte-cartouche, et
où les premier et second moyens de couplage sont configurés comme des moyens de couplage rotatif coopérant (345, 355, 445, 455) permettant à une cartouche d'être fixée au porte-cartouche par un mouvement de rotation relatif entre eux.

2. Système d'administration de médicament selon la revendication 1, dans lequel la cartouche comprend une interface d'aiguille (342) permettant à un assemblage d'aiguille d'être monté en communication fluidique avec le réservoir.

3. Système d'administration de médicament selon la revendication 2, dans lequel la cartouche comprend une partie de corps (310) formant la partie de corps cylindrique, l'interface d'aiguille (342) et le premier moyen de couplage (345) étant fixés sur la partie sortie distale.

4. Système d'administration de médicament selon la revendication 3, dans lequel la cartouche comprend une partie de couplage solidaire (340, 440, 540, 640) formant l'interface d'aiguille et le premier moyen de couplage.

5. Système d'administration de médicament selon la revendication 3, dans lequel la partie de corps comprend une partie col distale (313) avec une ouverture (311) fermée par un opercule perforable par une aiguille (330), la partie de couplage solidaire (340) étant fixée à la partie col.

6. Système d'administration de médicament selon la revendication 6, dans lequel l'opercule (330) est fixé à la partie col par un élément circonférentiel (331) auquel la partie de couplage solidaire est fixée.

7. Système d'administration de médicament selon l'une quelconque des revendications 2 à 6, dans lequel la partie de corps cylindrique, l'interface d'aiguille et le premier moyen de couplage sont formés intégralement à partir d'un matériau polymère.

8. Système d'administration de médicament selon l'une quelconque des revendications précédentes, dans lequel les premier et second moyens de couplage sont amovibles pour permettre à un utilisateur de retirer une cartouche montée du porte-cartouche.

9. Système d'administration de médicament selon l'une quelconque des revendications précédentes, comprenant en outre un boîtier (220) dans lequel l'assemblage d'expulsion est disposé au moins partiellement, le porte-cartouche (210) étant formé en tant que partie du boîtier.

10. Système d'administration de médicament selon l'une quelconque des revendications précédentes, dans lequel :
- le piston pouvant se déplacer axialement a une position proximale initiale, une position distale entièrement vers l'avant, et une position intermédiaire entre celles-ci,
- la tige de piston est mobile entre une position chargée proximale et une position la plus distale, une position de chargement étant définie de manière distale par rapport à la position proximale,
- la tige de piston, lorsqu'elle est positionnée à proximité de la position de chargement, est déplacée vers sa position de chargement lorsqu'une cartouche est retirée d'une position chargée, et
- la tige de piston est adaptée pour venir en prise avec un piston et être déplacée de manière proximale vers sa position de chargement lorsqu'une cartouche est disposée dans une position chargée.

11. Système d'administration de médicament selon l'une quelconque des revendications précédentes, comprenant un dispositif d'administration de médicament comprenant le porte-cartouche et l'assemblage d'expulsion.
